# EUROPEAN PATENT SPECIFICATION

(11) **EP 0 680 752 B1**
(45) Date of publication and mention of the grant of the patent: **14.11.2001**
(21) Application number: 95111178.0
(22) Date of filing: 24.03.1992
(51) Int. Cl.: A61K 9/14, A61K 31/13, A61K 9/72

(54) **Conditioned water soluble substances**
Konditionierte wasserlösliche Substanzen
Substances conditionnées, solubles dans l'eau

(30) Priority: 11.04.1991 SE 9101090
(43) Date of publication of application: 08.11.1995
(62) Divisional of application: 92907877.2
(73) Proprietor: AstraZeneca AB, 151 85 Södertälje (SE)
(72) Inventor: Byström, Katarina Ulla, S-24013 Genarp (SE); Jakupovic, Edib, S-15531 Nykvarn (SE); Trofast, Jan William, S-22647 Lund (SE); Trofast, Eva Ann-Christin, S-22647 Lund (SE)
(74) Representative: Linderoth, Margareta

(56) References cited:
- EP-A- 0 239 798
- EP-A- 0 499 299
- US-A- 4 866 051

## Description

### Field of the Invention

The present invention relates to water-soluble micronised substances, which can be produced, stored and used while maintaining the aerodynamic properties required for inhalation of such substances and which have improved physicochemical properties in the dry state, thereby facilitating the technical handling and significantly increasing the medical value of the substances.

### Background of the Invention

During the past few years, there have been frequent demonstrations of the fact that the appropriate selection of the most suitable crystalline modification significantly can influence the clinical results of a given chemical entity. The chemical and physical stability of a solid compound in a particular dosage form can be modified by presenting the substance in the appropriate crystal form. Little information is available on the role of polymorphism and crystal habit in solid dosage form and powder technology. It is, however, apparent that the appropriate selection of the most suitable crystalline modification, whether arising from polymorphic differences of as a result of solvate complex formation of both water-soluble substances and less water-soluble substances such as theophylline, often significantly can increase the medical value of a given drug in a particular dosage form. There are only a few statements available to predict the outcome of a crystallisation procedure if e.g. the substance could be involved in different polymorphic or pseudopolymorphic forms. Solid-state transformations may also occur during mechanical treatment, e.g. micronisation and by pressure during tableting. While a few generalisations can be made concerning the influence of structural modifications on the tendency of a chosen compound to exhibit polymorphism or other phenomena, a complete understanding of this problem awaits further research. Often "trial and error" approaches are used to develop a successful formulation of a drug. It is necessary to establish the conditions whereby different forms of a substance might be converted to a single form thus eliminating differences in solid-state properties and subsequent different physico-chemical properties.

E. Shefter and T. Higuchi have measured the relative rates of dissolution of several crystalline solvated and non-solvated forms of important pharmaceuticals, J. Pharm. Sci., 52 (8), (1963), 781-91.

L. van Campen, G. Zografi and J.T. Carstensen give in a review article an approach to the evaluation of hygroscopicity for pharmaceutical solids, Int. J. Pharmceut. 5, (1980), 1-18.

C. Ahlnech and G. Zografi describe the molecular basis of moisture on the physical and chemical stability of drugs in the solid state, Int. J. Pharmceut., 62, (1990), 87-95.

M. Otsuka et al. have calculated hydration data using various solid-state kinetic models for theophylline anhydrate powder, J. Pharm. Pharmacol., 42, (1990), 606-610.

Hak-Kim Chan and Igor Gonda have examined the properties of respirable crystals of cromoglycic acid by using different methods, J. Pharm. Sci., 78 (2), (1989), 176-80.

A more comprehensive discussion of factors relating to pharmaceutical preformulations and the physicochemical properties of drug substances is given by J.I. Wells in Pharmaceutical Preformulation: The Physicochemical Properties of Drug Substances, John Wiley & Sons, New York (1988). See particularly the chapter about polymorphism pp 86-91.

US 4, 866,051 discloses pharmaceutical powder compositions comprising micronised beclomethasone dipropionate monohydrate which are said to be stable after prolonged storage.

### Brief description of the Invention

The object of the invention is to provide water-soluble micronised substances, which can be produced, stored and used while maintaining the aerodynamic properties required for inhalation of such substances, whereby reducing the residual water from the micronised substances, conditioning said dried, micronised substances with a solvent and finally eliminating residual solvent from the substances.

### Detailed description of the Invention

In a first aspect the invention provides a water soluble micronised substance having a particle size of less than 10 µm in a stable crystalline form, wherein the particle size of the micronised substance is identical before and after conditioning said micronised substance with a solvent, characterised in that the substance is a β₂-adrenergic agonist selected from terbutaline or salbutamol sulphate.

Preferably the terbutaline sulphate of the invention has a surface area when measured by BET nitrogen adsorption using a Flowsorb II 2300 of from 7 to 9 m²/g after having been standing in high humidity for 24 hours.

Preferably the terbutaline sulphate has a heat of evolution when subjected to water vapour and when measured using a Thermometic 2277 Thermal Activity Monitor of 0.1 J/g.

Preferably the salbutamol sulphate according to the invention has a surface area when measured by BET nitrogen using a Flowsorb II 2300 of 5.9m²/g after having been standing in high humidity for 24 hours.

Preferably the salbutamol sulphate according to the invention has a heat of evolution when subjected to water vapour and when measure using a Thermometric 2277 Thermal Activity Monitor of 0.1 J/G.

The water soluble micronised substances of the present invention can be obtained by a reliable process, where the desired polymorphic form can be conveniently and reproducibly prepared. It relates to a three-step procedure: -
a. reducing, if necessary, the residual water from the micronised substance by drying optionally at an elevated temperature and/or vacuum.
b. conditioning said dried micronised substance with a solvent, and
c. eliminating the residual solvent by storing the substance in a dry place, such as vacuum, or by purging with an inert gas.

The solvents used in the conditioning step b) are organic alcohols, ketones, esters, acetonitrile and the like, most preferably lower alcohols like methanol, ethanol, n-propanol, isopropanol; lower ketones like acetone, methylethylketone, ethylacetate, preferably in the vapour phase.

According the one preferred embodiment the conditioning step b) is carried out in an inert gas containing solvent vapour.

The inert gas used in step c) and optionally in step b) is preferably nitrogen.

The preferred substances on which the invention is to be applied are terbutaline sulphate and salbutamol sulphate.

Terbutaline sulphate and salbutamol sulphate, are highly selective β₂-adrenergic agonist having bronchospasmolytic effect and are effective in the treatment of reversible obstructive lung ailments of various genesis, particularly asthmatic conditions. Disodium chromoglycate (DSCG) has been used as a prophylactic agent in the treatment of allergic bronchial asthma for many years.

The invention will be described by using terbutaline sulphate and salbutamol sulphate. The phenomena of solvate formation and polymorphism are well recognised in the literature in the preformulation studies in the development phase for new drugs in the solid state. e.g. the US Pharmacopoeia recognises <90 drug hydrates!

Many substances exist in different polymorphs (pseudopolymorphs) and several metastable solvates with variable composition and physical properties like bulk density and hygroscopicity. Several transformations between these polymorphs may occur at different velocity. These effects are operating when crystalline substances have been activated by various processes such as grinding, freeze drying, micronisation or recrystallisation to produce regions of partial amorphous structure. The substances often will be obtained in an amorphous state or a metastable crystalline form when spray drying, freeze drying, rapid solvent quenching or when using controlled precipitation where both crystalline and amorphous forms can be prepared. The use of an amorphous form or a metastable crystalline form is often limited due to its thermodynamic instability. It is therefore a desire to convert the amorphous form or the metastable crystalline state. The present invention deals with such physical and chemical changes, or more importantly, to anticipate them and the means by which these solid-state phenomena can be handled.

After recrystallisation (or after spray drying/freeze drying) the substance has to be micronised to the final particle size required for e.g. inhalation. The particles is less than 10 µm. For crystalline substances, the micronisation step seems to give an amorphous outer layer of the particle making the particle more sensitive to moisture.

It is an object of this invention to be able to reliably provide a crystalline form of certain water-soluble substances, which can be produced, stored and used, while maintaining the aerodynamic properties and specifications (particle size, particle form, hydroscopicity etc.) required for inhalation of such substances. The particle size of the micronised substances is identical before and after the conditioning step as measured by different instruments like Malvern Master Sizer, culter counter or a microscope.

The condition of the substance probably rearrange the outer layer of the crystals of the amorphous substance giving a more stable and less hygroscopic product.

In some instances it has been possible to use infrared spectroscopy in order to study the conversion of an amorphous form or a partly crystalline form into a stable crystalline form. Other methods available include BET gas adsorption, X-ray powder diffraction, microcalorimetry and differential scanning calorimetry (DSC). We have found that BET gas adsorption and microcalorimetry being the best methods for distinguishing the different forms of the tested compounds.

### Test Results

The surface area measured by determining the quantity of a gas (nitrogen) that adsorbs as a single layer of molecules, a monomolecular layer on a sample is formed (Flowsorb II 2300, Micromeritics Co., USA). Surface area after the sample has. been standing in high humidity for 24 hours.

| Micronised substance (m²/g) | Non-conditioned substance (m²/g) | Conditioned substance (m²/g) |
|---|---|---|
| Terbutaline sulphate: | | |
| 11-12.5 | <3 | 7 - 9 |

| Salbutamol sulphate: | | |
|---|---|---|
| 8.4 | 3 | 5.9 |

With low surface area, obtained when micronised substance has been stored at high humidity, the bulk substance has a great tendency to aggregate when stored, which makes the substance very difficult for technical handling in manufacturing the different formulations needed.

The interactions between certain substances and water vapour have also been studied by microcalorimetry. When said substances are subjected to water in the vapour phase they give off heat in a highly co-operative process. This moisture induced phase transition is however not observed for the conditioned substance. Thus, the conditioning process transforms the substance into a more stable form that is less sensitive to humidity.

Comparison of the heat given off by non-conditioned and conditioned substances when subjected to water vapour. Experiments are performed by a Thermal Activity Monitor 2277 (Thermometrics, Sweden).

| | Heat (J/g) | |
|---|---|---|
| Relative humidity (%) | Non-conditioned substance | Conditioned substance |
| Terbutaline sulphate | | |
| 58 | 3.6 | 0.1 |
| 75 | 6.2 | 0.1 |

| Salbutamol sulphate | | |
|---|---|---|
| 75 | 6-8 | 0.1 |

The stability of the particles being conditioned were astonishing and will in a remarkable way increase the flexibility of the use of the substance for different formulations.

### Experimental procedure

The invention is further illustrated but not limited by the following example.

### Example 1

3.6 kg terbutaline sulphate micronised was dried in a stainless steel column with 200 mm diameter at 90°C in vacuum for 23 hours. The dried substance was cooled to about 30°C and the pressure was normalised with ethanol-saturated nitrogen gas. 70 ml/min of ethanol-saturated nitrogen gas was then passed through the 200 mm diameter column for 60 hours to condition the substance. During this time the column was inverted a few times. The residual solvent was eliminated by purging with nitrogen gas for 2 hours and the product, about 3.5 kg. was packed in double plastic bags with a drying agent between the bags.

### Example 2

In one experiment 1 g micronised salbutamol sulphate was kept at room temperature for 24 hours in a closed vessel containing a beaker filled with ethanol. The sample was removed and stored in a completely dry environment over night in order to eliminate traces of ethanol. The sample was subjected for analysis (see test results given above).

It is necessary to introduce stirring or tumbling of the substance when conditioning in larger scale.

## Claims

1. A water soluble micronised substance having a particle size of less than 10 µm in a stable crystalline form, wherein the particle size of the micronised substance is identical before and after conditioning said micronised substance with a solvent, **characterised in that** the substance is a β₂-adrenergic agonist. selected from terbutaline or salbutamol sulphate.

2. Terbutaline sulphate according to claim 1, which has a surface area when measured by BET nitrogen adsorption using a Flowsorb II 2300 of from 7 to 9 m²/g after having been standing in high humidity for 24 hours.

3. Terbutaline sulphate according to either claim 1 or claim 2 which has a heat evolution when subjected to water vapour and when measured using the Thermometric 2277 Thermal Activity Monitor of 0.1 J/g.

4. Salbutamol sulphate according to claim 1, which has a surface area when measured by BET nitrogen using a Flowsorb II 2300 of 5.9m²/g after having been standing in high humidity for 24 hours.

5. Salbutamol sulphate according to either claim 1 or 4, which has a heat of evolution when subjected to water vapour and when measured using a Thermometric 2277 Thermal Activity Monitor of 0.1 J/G.

## Patentansprüche

1. Wasserlösliche, mikronisierte Substanz mit einer Teilchengröße von weniger als 10 *µ*m in stabiler kristalliner Form, wobei die Teilchengröße der mikronisierten Substanz vor und nach der Konditionierung der mikronisierten Substanz mit einem Lösungsmittel identisch ist, **dadurch gekennzeichnet, daß** es sich bei der Substanz um einen β₂-adrenergen Agonisten aus der Gruppe Terbutalin- und Salbutamolsulfat handelt.

2. Terbutalinsulfat nach Anspruch 1, das nach 24stündigem Stehenlassen in einer Atmosphäre mit hoher Luftfeuchtigkeit eine durch BET-Stickstoff-Adsorption unter Verwendung eines Flowsorb II 2300 gemessene Oberfläche von 7 bis 9 m²/g aufweist.

3. Terbutalinsulfat nach Anspruch 1 oder 2, das bei Einwirkung von Wasserdampf 0,1 J/g Wärme abgibt, gemessen mit einem Thermometric 2277 Thermal Activity Monitor.

4. Salbutamolsulfat nach Anspruch 1, das nach 24stündigem Stehenlassen in einer Atmosphäre mit hoher Luftfeuchtigkeit eine durch die BET-Stickstoff-Methode unter Verwendung eines Flowsorb II 2300 bestimmte Oberfläche von 5,9 m²/g aufweist.

5. Salbutamolsulfat nach Anspruch 1 oder 4, das bei Einwirkung von Wasserdampf 0,1 J/g Wärme abgibt, gemessen mit einem Thermometric 2277 Thermal Activity Monitor.

## Revendications

1. Substance micronisée soluble dans l'eau ayant une taille particulaire de moins de 10 µm dans une forme cristalline stable, dans laquelle la taille particulaire de la substance micronisée est identique avant et après un conditionnement de ladite substance micronisée avec un solvant, **caractérisée en ce que** la substance est un agoniste β₂-adrénergique choisi parmi le sulfate de terbutaline ou de salbutamol.

2. Sulfate de terbutaline suivant la revendication 1, qui a une aire superficielle de 7 à 9 m²/g lorsque mesurée par adsorption d'azote BET en utilisant un Flowsorb II 2300 après avoir séjourné en humidité élevée pendant 24 heures.

3. Sulfate de terbutaline suivant la revendication 1 ou la revendication 2, qui présente un dégagement de chaleur de 0,1 J/g lorsqu'il est soumis à de la vapeur d'eau et lorsque mesuré en utilisant l'enregistreur d'activité thermique Thermometric 2277.

4. Sulfate de salbutamol suivant la revendication 1, qui a une aire superficielle de 5,9 m²/g lorsque mesurée par adsorption d'azote BET en utilisant un Flowsorb II 2300 après avoir séjourné en humidité élevée pendant 24 heures.

5. Sulfate de salbutamol suivant la revendication 1 ou 4, qui présente un dégagement de chaleur de 0,1 J/g lorsqu'il est soumis à de la vapeur d'eau et lorsque mesuré en utilisant l'enregistreur d'activité thermique Thermometric 2277.
